# EUROPEAN PATENT SPECIFICATION

(11) **EP 2 657 340 B1**
(45) Date of publication and mention of the grant of the patent: **16.11.2016**
(21) Application number: 12002914.5
(22) Date of filing: 25.04.2012
(51) Int. Cl.: C12N 15/113, C07K 14/47, C07K 16/18

(54) **Inhibitors of thioredoxin-interacting protein (TXNIP) for therapy**
Hemmer von mit Thioredoxin interagierendem Protein (TXNIP) zur Therapie
Inhibiteurs de la protéine d'interaction de thiorédoxine (TXNIP) pour thérapie

(43) Date of publication of application: 30.10.2013
(73) Proprietor: Deutsches Krebsforschungszentrum, 69120 Heidelberg (DE)
(72) Inventor: Krammer, Peter, Prof. Dr., 69120 Heidelberg (DE); Gülow Karsten, Dr., 69123 Heidelberg (DE); Sass, Sabine, 69124 Heidelberg (DE)
(74) Representative: Schüssler, Andrea

(56) References cited:
- US-A1- 2010 272 710
- JI-HONG LIU ET AL: "Possible role for the thioredoxin system in the protective effects of probucol in the pancreatic islets of diabetic rats", CLINICAL AND EXPERIMENTAL PHARMACOLOGY AND PHYSIOLOGY, vol. 38, no. 8, 1 August 2011 (2011-08-01) , pages 528-533, XP55037317, ISSN: 0305-1870, DOI: 10.1111/j.1440-1681.2011.05545.x
- MOHAMMED MH AL-GAYYAR ET AL: "Thioredoxin interacting protein is a novel mediator of retinal inflammation and neurotoxicity", BRITISH JOURNAL OF PHARMACOLOGY, vol. 164, no. 1, 1 September 2011 (2011-09-01), pages 170-180, XP55037316, ISSN: 0007-1188, DOI: 10.1111/j.1476-5381.2011.01336.x
- S. M. TAN ET AL: "Tranilast attenuates the up-regulation of thioredoxin-interacting protein and oxidative stress in an experimental model of diabetic nephropathy", NEPHROLOGY DIALYSIS TRANSPLANTATION, vol. 26, no. 1, 1 January 2011 (2011-01-01), pages 100-110, XP55037382, ISSN: 0931-0509, DOI: 10.1093/ndt/gfq355
- SHAKED M ET AL: "Insulin counteracts glucotoxic effects by suppressing thioredoxin-interacting protein production in INS-1E beta cells and in Psammomys obesus pancreatic islets", DIABETOLOGIA ; CLINICAL AND EXPERIMENTAL DIABETES AND METABOLISM, SPRINGER, BERLIN, DE, vol. 52, no. 4, 13 February 2009 (2009-02-13), pages 636-644, XP019698549, ISSN: 1432-0428
- B. J. DEROO: "Estradiol Regulates the Thioredoxin Antioxidant System in the Mouse Uterus", ENDOCRINOLOGY, vol. 145, no. 12, 1 January 2004 (2004-01-01), pages 5485-5492, XP55037445, ISSN: 0013-7227, DOI: 10.1210/en.2004-0471
- WATANABE R ET AL: "Anti-oxidative, anti-cancer and anti-inflammatory actions by thioredoxin 1 and thioredoxin-binding protein-2", PHARMACOLOGY AND THERAPEUTICS, ELSEVIER, GB, vol. 127, no. 3, 1 September 2010 (2010-09-01), pages 261-270, XP027120391, ISSN: 0163-7258 [retrieved on 2010-07-03]

## Description

The present invention provides a compound capable of reducing or inhibiting (a) the biological activity of thioredoxin-interacting protein (TXNIP) or (b) the expression of the gene encoding TXNIP for use in a method of improving female fertility.

Life extension science, also known as anti-aging medicine, experimental gerontology, and biomedical gerontology, is the study of slowing down or reversing the processes of aging to extend both the maximum and average lifespan, including prolonging and improving female fertility. Some researchers in this area, and "life extensionists" or "longevists" believe that future breakthroughs in tissue rejuvenation with stem cells, molecular repair, and organ replacement (such as with artificial organs or xenotransplantations) will eventually enable humans to have indefinite lifespans and fertility spans through complete rejuvenation to a healthy youthful condition. The sale of putative anti-aging products such as nutrition, physical fitness, skin care, hormone replacements, vitamins, supplements and herbs is a lucrative global industry, with the US market generating about $50 billion of revenue each year. However, medical experts state that the use of such products has not been shown to affect the aging process, and many claims of anti-aging medicine advocates have been roundly criticized by medical experts, including the American Medical Association.

Deroo et al., Endocrinology, Vol. 145, No. 12, pp. 5485-5492 (2004) describe that the thioredoxin antioxidant system in the mouse uterus is regulated by estradiol.

Thus, the technical problem underlying the present invention is to provide means for extending both the maximum and average fertility span.

The solution of said technical problem is achieved by providing the embodiments characterized in the claims.

In particular, the present invention concerns a compound capable of reducing or inhibiting (a) the biological activity of thioredoxin-interacting protein (TXNIP) or (b) the expression of the gene encoding TXNIP for use in a method of improving female fertility,
characterized in that said compound is
(i) an antisense oligonucleotide or shRNA reducing or inhibiting the expression of the gene encoding TXNIP, or
(ii) an antibody directed against TXNIP or a fragment thereof, or
(iii) an inactive version of TXNIP or nucleic acid sequences encoding an inactive version of TXNIP.

In addition, the present invention concerns a method for identifying a compound that improves female fertility by reducing or inhibiting the biological activity of TXNIP or the expression of the gene encoding TXNIP, comprising the steps of:
(a) incubating a candidate compound with a test system comprising TXNIP or the gene encoding TXNIP; and
(b) assaying a biological activity of TXNIP;
wherein an inhibition or loss of the biological activity of TXNIP is indicative of the presence of a candidate compound having the desired property.

One of the major factors contributing to aging and loss of female fertility (which is viewed by many scientists as a "disease") and malfunctioning of an organism is the accumulation of oxidative damage in cellular macromolecules that contributes to loss of function and higher mortality in the aged population (1). During the experiments resulting in the present invention it was found that thioredoxin-interacting protein (TXNIP) negatively influences the redox balance and cellular defense capacity during aging. Thioredoxin-interacting protein (TXNIP), also known as VDUP1 (Vitamin D₃ upregulated protein 1) or TBP-2 (thioredoxin-binding protein 2) was originally identified as a protein that is upregulated upon vitamin D₃ treatment of HL-60 leukemia cells (3,4). TXNIP is a major regulator of the cellular redox state by interacting with and thereby inhibiting the anti-oxidative function of thioredoxin (TXN) (5,6). In addition, TXNIP has been demonstrated to play a role in various pathological conditions, such as metabolic, cardiac and inflammatory diseases as well as cancer. TXNIP is considered as a tumor suppressor by either inhibition of proliferation and/or induction of apoptosis (4, 7-9). A prominent role for TXNIP was also shown in the development of diabetes. It was found to be overexpressed in pre-diabetic and diabetic patients, and lack of TXNIP expression was associated with improved β-cell function and glucose tolerance (8,10). In addition, glucose-induced TXNIP expression was implicated in the activation of the inflammasome and induced the secretion of the pro-inflammatory cytokine interleukin-1β and, thereby, promoted β-cell death (11).

The inventors found that expression of TXNIP is greatly enhanced in various human cell types of aged individuals which leads to decreased stress resistance towards oxidative stress. Significantly, disruption of TXNIP in *Drosophila melanogaster* increased thioredoxin (TXN) anti-oxidative activity and thereby improved the resistance to starvation and oxidative stress. Remarkably, TXNIP deficiency also significantly prolonged healthy lifespan and at the same time enhanced egg laying. The results of the present invention indicate that TXNIP deficiency extends healthy lifespan and positively influences female fertility again proving that longevity extension and fecundity are not regulated exclusively in an opposite way (2). The data further suggests that enhanced TXNIP expression during aging might represent a universal mechanism that affects lifespan, stress resistance, and diseases in the elderly.

In summary, the present invention describes a novel role for the redox regulator TXNIP in the control of healthy lifespan and resistance to oxidative stress. Without wishing to be bound by theory it is thought that TXNIP decreases TXN availability and activity and thereby impairs cellular redox homeostasis. Since oxidative stress is one of the major factors influencing damage accumulation and cellular deterioration during the aging process, counterregulation of TXNIP expression would allow to improve cellular defense capacity (1,25). Alternatively, the results of the present invention do not exclude the possibility that enhanced TXNIP expression during aging may also influence non-redox dependent TXNIP functions. Thereby it could also affect the development of age-related diseases and deterioration of cellular function. The findings that TXNIP is enhanced during aging connect well to the increase in age-related diseases in the elderly, such as diabetes. In concurrence, enhanced TXNIP expression could also be shown in rheumatoid arthritis patients. It is conceivable that enhanced expression of TXNIP that was observed in various human tissues during aging will contribute to deterioration of various age-related diseases. Thus, healthy lifespan may be increased by antagonizing TXNIP upregulation during aging.

### Brief description of the drawings

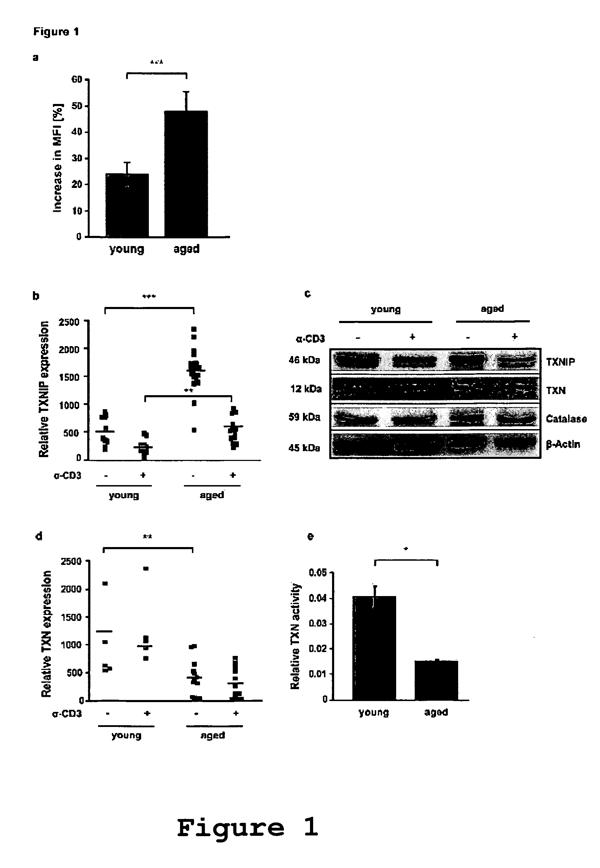

Figure 1: Enhanced ROS generation in T cells of aged individuals.
   T cells were isolated from blood of young (20 - 25 years old) and aged donors (> 55 years old).
   **a:** T cells from aged donors show enhanced activation-induced ROS generation. Primary T cells were stained with Dichlorodihydrofluorescein diacetate (H₂DCF-DA) and increase in "Mean Fluorescence Intensity" (MFI) was analyzed by FACS (n = 11 young donors and 16 aged donors, respectively, p < 0.001).
   **b, c:** T cells of aged individuals show increased TXNIP expression in unstimulated cells as well as upon TCR triggering as determined by (**b**) qPCR (p < 0.001) or (**c**) Western blotting.
   **d:** TXN expression is slightly decreased in T cells of aged individuals as determined by qPCR (n = 5 young donors and n = 13 aged donors, respectively, p < 0.01).
   **e:** TXN activity is decreased in T cells of aged individuals as determined by TXN activity assay (p < 0.01).

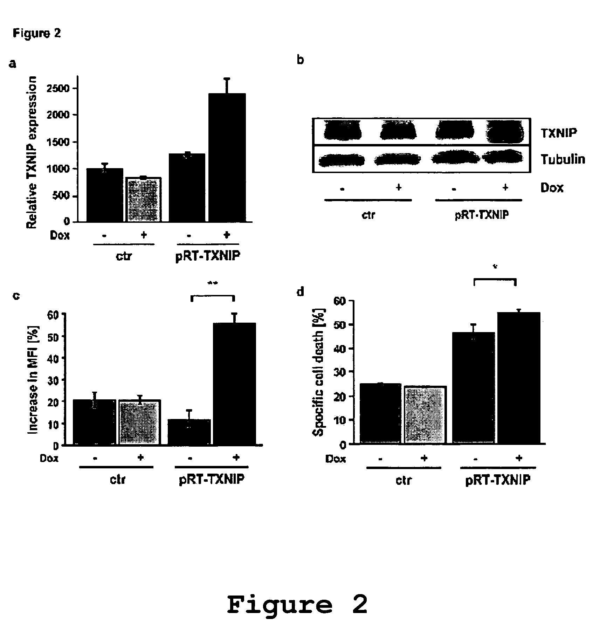

Figure 2: TXNIP upregulation enhances ROS generation and susceptibility to cell death
   **a, b:** Jurkat cells stably transduced with an inducible expression vector for TXNIP or a control vector (ctr) were either left untreated or treated with 1 µg/ml Dox for 24 hours and TXNIP expression was analyzed by (**a**) qPCR, or (**b**) Western blotting.
   **c:** TXNIP or ctr expression were either induced by Dox treatment for 24 hours or cells were left untreated. Afterwards, cells were stained with DCF and ROS release was analyzed by flow cytometry (p = 0.001).
   **d:** Cell death was assessed by FSC/SSC measurement by flow cytometry upon stimulation with plate-bound α-CD3 antibodies for 24 hours (p = 0.02). Data is representative of at least three independent experiments.

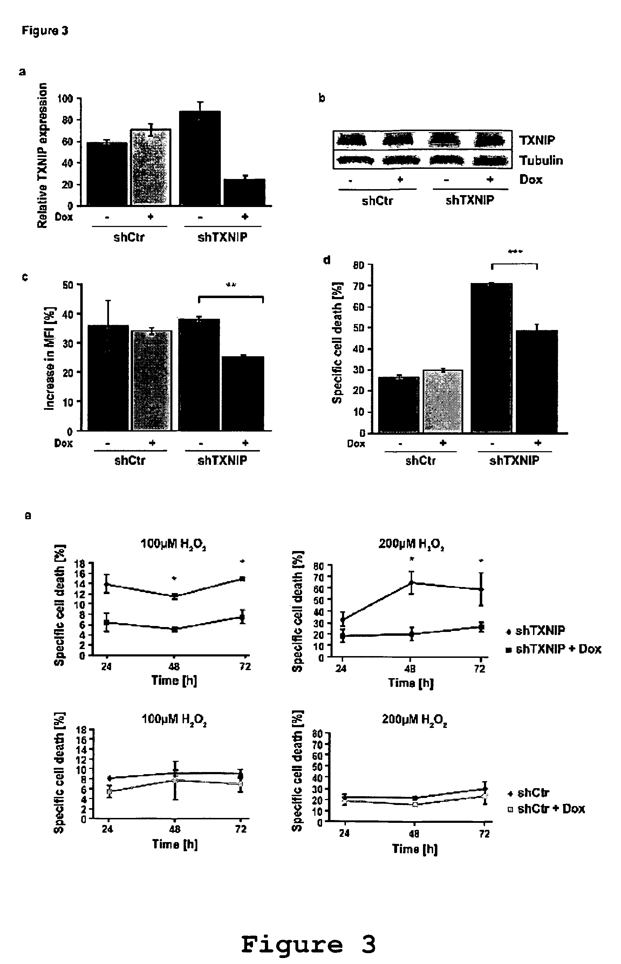

Figure 3: TXNIP downregulation reduces ROS generation and enhances stress resistance.
   **a, b:** Jurkat cells were stably transduced with an inducible shRNA construct against TXNIP or a scrambled control (shCtr). Transduced cells were incubated with 1 µg/ml Dox for 24 hours and TXNIP mRNA expression was analyzed by (**a**) qPCR and (**b**) TXNIP protein levels by Western blot, respectively.
   **c:** Stably transduced Jurkat cells were either left untreated or treated with Dox for 24 hours and subsequently stimulated with plate-bound α-CD3 antibodies for 1 hour. Afterwards, cells were stained with DCF and ROS release analyzed by flow cytometry (p = 0.006).
   **d:** Cell death was assessed by FSC/SSC measurement by flow cytometry upon stimulation with plate-bound α-CD3 antibodies for 24 hours (p < 0.001).
   **e:** Jurkat T cells were stably transduced with an shRNA against TXNIP and its expression was induced by Dox treatment (+ Dox) for 24 hours or cells were left untreated. Afterwards, cell death was induced by treatment with 100 or 200 µM H₂O₂ and cell death was analyzed by flow cytometry at the indicated time points. Data is representative of at least three independent experiments.

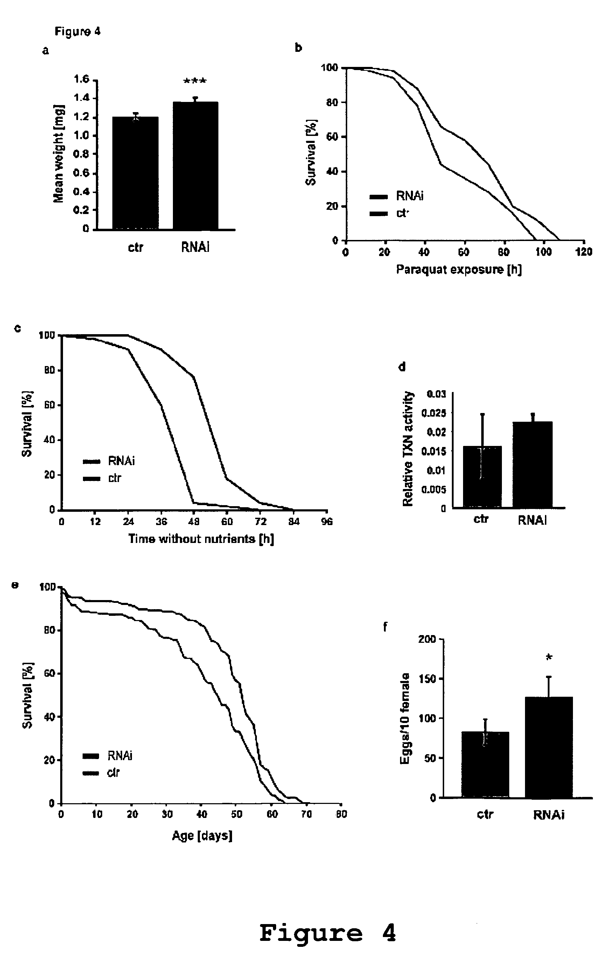

Figure 4: TXNIP deficient flies show lifespan extension, enhanced stress resistance and increased female fertility.
   **a:** Increased body weight of TXNIP deficient female adult flies at 1 day of age (mean ± SEM; n = 40 flies per group).
   **b:** TXNIP deficient flies show extended lifespan. Survival curves of ctr and RNAi flies (p < 0.001, n = 200 flies per group).
   **c:** TXNIP deficiency enhances oxidative stress resistance. Survival curves of flies exposed to 1 mM paraquat for the indicated times (n = 50 flies per group).
   **d:** TXNIP deficient flies show enhanced starvation stress resistance. Survival curves of starved flies (n = 50 flies per group).
   **e:** Enhanced TXN activity in TXNIP deficient flies. Whole-fly protein lysates were subjected to TXN activity assay (n = 8 flies per group, p = 0.01).
   **f:** TXNIP deficient flies show enhanced fertility. Data is presented as mean number of eggs laid per female per day (± SD, p = 0.01). Eggs from 5 vials per group containing ten flies per vial were counted. Experiments were repeated independently three times. A representative experiment is shown.
   For all survival experiments one representative experiment out of at least three independent experiments is shown.

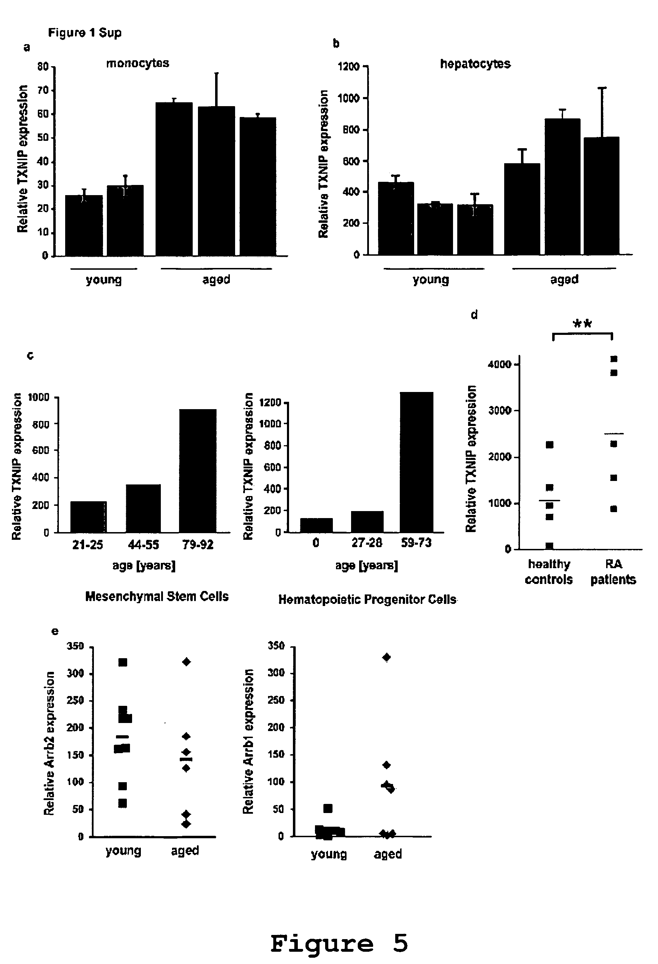

Figure 5: TXNIP expression is enhanced in a variety of cell types
   **a:** Monocytes of aged blood donors show enhanced TXNIP mRNA expression as determined by qPCR (p = 0.001).
   **b:** mRNA isolated from young and aged hepatocytes reveals enhanced TXNIP expression in hepatocytes from aged donors.
   **c:** Affymetrix gene chip array analysis reveals higher TXNIP expression in mesenchymal stem cells and hematopoietic progenitor cells isolated from aged donors.
   **d:** MACS isolated CD4⁺ T cells of rheumatoid arthritis patients show enhanced TXNIP expression analyzed by qPCR (p = 0.04).
   **e:** Arrestin family members ARRB1 and ARRB2 show similar TXNIP mRNA expression in T cells of young (n = 8) and aged blood donors (n = 7) as determined by qPCR (p = 0.072 and 0.43, respectively).

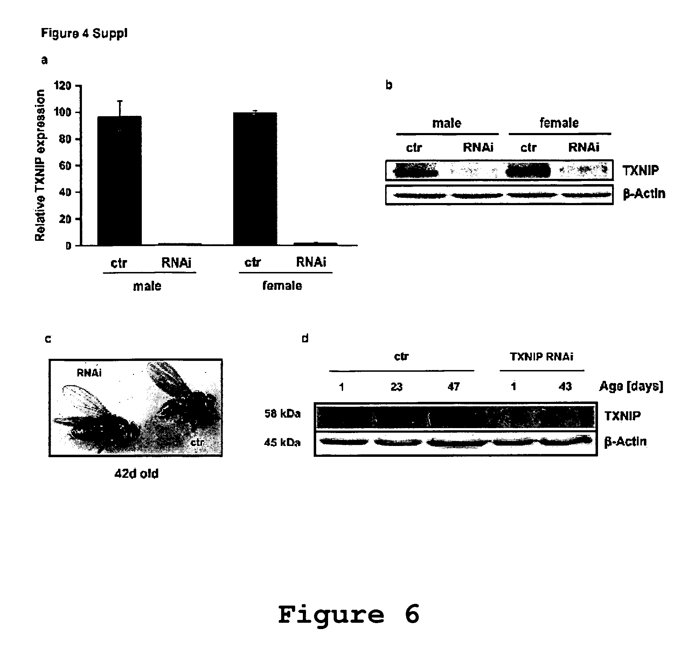

Figure 6: Higher TXNIP expression in aged flies
   **a, b:** TXNIP RNAi flies express undetectable levels of TXNIP as determined by either (**a**) qPCR or (**b**) Western blotting. Total RNA was extracted from the whole body of TXNIP RNAi (RNAi) and control (ctr) adult flies and gene expression analyzed by qPCR. Whole-fly protein lysates were prepared from RNAi or ctr flies and immunoblotted against TXNIP or β-actin.
   **c:** Appearance and body size of female adult RNAi and ctr flies, respectively.
   **d:** Whole-fly extracts were prepared from female adult flies at the indicated age and immunoblotted_with_antibodies against TXNIP and β-actin, respectively.

Thus, the present invention relates to a a compound capable of reducing or inhibiting (a) the biological activity of thioredoxin-interacting protein (TXNIP) or (b) the expression of the gene encoding TXNIP for use in a method of improving female fertility,
characterized in that said compound is
(i) an antisense oligonucleotide or shRNA reducing or inhibiting the expression of the gene encoding TXNIP, or
(ii) an antibody directed against TXNIP or a fragment thereof, or
(iii) an inactive version of TXNIP or nucleic acid sequences encoding an inactive version of TXNIP.

Inj addition, the present invention relates to a method for identifying a compound that improves female fertility by reducing or inhibiting the biological activity of TXNIP or the expression of the gene encoding TXNIP, comprising the steps of:
(a) incubating a candidate compound with a test system comprising TXNIP or the gene encoding TXNIP; and
(b) assaying a biological activity of TXNIP;
wherein an inhibition or loss of the biological activity of TXNIP is indicative of the presence of a candidate compound having the desired property.

As used herein, "oxidative stress" means an imbalance between the production and manifestation of reactive oxygen species and a biological system's ability to readily detoxify the reactive intermediates or to repair the resulting damage. Disturbances in the normal redox state of tissues can cause toxic effects through the production of peroxides and free radicals that damage all components of the cell, including proteins, lipids, and DNA. Some reactive oxidative species can even act as messengers through a phenomenon called redox signaling. In humans, oxidative stress is involved in many diseases. Examples include: Sickle Cell Disease, atherosclerosis, Parkinson's disease, Alzheimer's disease, heart failure, myocardial infarction, Schizophrenia, Bipolar disorder, fragile X syndrome and chronic fatigue syndrome. Short-term oxidative stress may also be important in prevention of aging by induction of a process named mitohormesis.

The reduction or inhibition of the biological activity can be effected by direct interaction or binding of a compound to TXNIP or by indirect interaction, e.g., by interacting with a compound that is associated with the biological activity of TXNIP. The reduction or inhibition of the biological activity can also be achieved by the application of altered, e.g., inactive forms of TXNIP, preferably in excess.

Examples of suitable compounds reducing or inhibiting the biological activity of TXNIP or the expression of the gene encoding TXNIP with the aim to get a therapeutic effect are:
(a) Plasmids, vectors or natural/synthetic/mutated viruses, oligonucleotides of various types of modification (e.g. PTO, LNA, 2'F-ANA, protein-nucleotide complexes, shRNA ("small hairpin RNA" or "short hairpin RNA" which is a sequence of RNA that makes a tight hairpin turn that can be used to silence gene expression via RNA interference), RNA*ᵢ*, siRNA or mikroₘᵢRNA, Methylmetoxy-, Phosphoroamidates, PNA, Morpholino, Phosphoramidate, Cyclohexen (CeNA), gap-meres, ribozymes, aptamers, CpG-oligos, DNA-zymes, riboswitches, or lipids or lipid containing molecules;
(b) peptides, peptide complexes, including all types of linkers,
(c) small molecules;
(d) antibodies and their derivatives, especially chimeras, Fab-fragments, Fc-fragments;
(e) carriers, liposomes, nanoparticles, complexes, or any other delivery systems containing the above named constructs; or
(f) oxidizing agents or sulfhydryl (SH groups) modifying agents.

Further compounds suitable for the purposes of the present invention and methods how to identify/select such compounds are in more detail described below.

Preferably, in a pharmaceutical composition, such compounds as described above are combined with a pharmaceutically acceptable carrier. "Pharmaceutically acceptable" is meant to encompass any carrier, which does not interfere with the effectiveness of the biological activity of the active ingredient and that is not toxic to the host to which it is administered. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and the active compound can be administered to the subject at an effective dose.

An "effective dose" refers to an amount of the active ingredient that is sufficient to affect the desired treatment. An "effective dose" useful for treatment may be determined using methods known to one skilled in the art (see for example (12)).

Administration of the suitable compositions may be effected by different ways, e.g. by intravenous, intraperitoneal, subcutaneous, intramuscular, topical or intradermal administration. The route of administration, of course, depends on the kind of therapy and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind of therapy, general health and other drugs being administered concurrently.

The person skilled in the art can easily identify or generate compounds useful for the treatments of the present invention based on the knowledge of the amino acid sequence of TXNIP, and the nucleotide sequence of the gene encoding this protein. Respective sequences are found in the NCBI data base (NCBI Reference Sequence: NP 006463.3)

According to the present invention, the compound useful for reducing or inhibiting the expression of the gene encoding TXNIP is an antisense oligonucleotide, shRNA or siRNA specific for TXNIP.

The generation of suitable antisense oligonucleotides includes determination of a site or sites within the TXNIP encoding gene for the antisense interaction to occur such that the desired effect, e.g., inhibition of the expression of the protein, will result. A preferred intragenic site is (a) the region encompassing the translation initiation or termination codon of the open reading frame (ORF) of the gene or (b) a region of the mRNA which is a "loop" or "bulge", i.e., not part of a secondary structure. If one or more target sites have been identified, oligonucleotides are chosen which are sufficiently complementary to the target, i.e., hybridize sufficiently well and with sufficient specificity, to give the desired effect. In the context of this invention, "hybridization" means hydrogen bonding, which may be Watson-Crick, Hoogsteen or reversed Hoogsteen hydrogen bonding, between complementary nucleoside or nucleotide bases. "Complementary" as used herein, refers to the capacity for precise pairing between two nucleotides. For example, if a nucleotide at a certain position of an oligonucleotide is capable of hydrogen bonding with a nucleotide at the same position of a DNA or RNA molecule, then the oligonucleotide and the DNA or RNA are considered to be complementary to each other at that position. The oligonucleotide and the DNA or RNA are complementary to each other when a sufficient number of corresponding positions in each molecule are occupied by nucleotides which can make hydrogen bonds with each other. Thus, "specifically hybridizable" and "complementary" are terms which are used to indicate a sufficient degree of complementarity or precise pairing such that stable and specific binding occurs between the oligonucleotide and the DNA or RNA target. It is understood in the art that the sequence of an antisense compound does not need to be 100% complementary to that of its target nucleic acid to be specifically hybridizable. An antisense compound is specifically hybridizable when binding of the compound to the target DNA or RNA molecule interferes with the normal function of the target DNA or RNA to cause a loss of utility, and there is a sufficient degree of complementarity to avoid non-specific binding of the antisense compound to non-target sequences under conditions in which specific binding is desired, i.e., in the case of therapeutic treatment.

The skilled person can generate antisense compounds, shRNAs and siRNAs according to the present invention on the basis of the known DNA sequence for TXNIP.

"Oligonucleotide" refers to an oligomer or polymer of ribonucleic acid (RNA) or deoxyribonucleic acid (DNA) or mimetics thereof. This term includes oligonucleotides composed of naturally-occurring nucleobases, sugars and covalent internucleoside (backbone) linkages as well as oligonucleotides having non-naturally-occurring portions which function similarly. Such modified or substituted oligonucleotides are often preferred over native forms because of desirable properties such as, for example, enhanced cellular uptake, enhanced affinity for nucleic acid target and increased stability in the presence of nucleases. While antisense oligonucleotides are a preferred form of the antisense compound, the present invention comprehends other oligomeric antisense compounds, including but not limited to oligonucleotide mimetics such as are described below. The antisense compounds in accordance with this invention comprise from about 8 to about 50 nucleobases (i.e. from about 8 to about 50 linked nucleosides). Particularly preferred antisense compounds are antisense oligonucleotides, even more preferably those comprising from about 15 to about 25 nucleobases. Antisense compounds include ribozymes, external guide sequences (EGS), oligonucleotides (oligozymes), and other short catalytic RNAs or catalytic oligonucleotides which hybridize to the target nucleic acid and inhibit its expression.

Alternatively, the compound of the invention is a vector allowing to transcribe an antisense oligonucleotide of the invention, e.g., in a mammalian host. Preferably, such a vector is a vector useful for gene therapy. Preferred vectors useful for gene therapy are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957.

In cases where expression should only occur in a particular target organ, the DNA sequences for transcription of the antisense oligonucleotides can be linked to a tissue specific promoter and used for gene therapy. Such promoters are well known to those skilled in the art (see e.g. (26-29)).

Within an oligonucleotide structure, the phosphate groups are commonly referred to as forming the internucleoside backbone of the oligonucleotide. The normal linkage or backbone of RNA and DNA is a 3' to 5' phosphodiester linkage. Specific examples of preferred antisense compounds useful in the present invention include oligonucleotides containing modified backbones or non-natural internucleoside linkages. Oligonucleotides having modified backbones include those that retain a phosphorus atom in the backbone and those that do not have a phosphorus atom in the backbone. Modified oligonucleotide backbones which can result in increased stability are known to the person skilled in the art, preferably such modification is a phosphorothioate linkage.

A preferred oligonucleotide mimetic is an oligonucleotide mimetic that has been shown to have excellent hybridization properties, and is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone (see e.g. (30).

Modified oligonucleotides may also contain one or more substituted or modified sugar moieties. Preferred oligonucleotides comprise one of the following at the 2' position: OH; F; 0-, S-, or N-alkyl; 0-, S-, or N-alkenyl; 0-, S- or N-alkynyl; or O-alkyl-O-alkyl, wherein the alkyl, alkenyl and alkynyl may be substituted or unsubstituted C₁ to C₁₀ alkyl or C₂ to C₁₀ alkenyl and alkynyl. A particularly preferred modified sugar moiety is a 2'-O-methoxyethyl sugar moiety.

Oligonucleotides of the invention may also include nucleobase modifications or substitutions. Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine etc., with 5-methylcytosine substitutions being preferred since these modifications have been shown to increase nucleic acid duplex stability.

Another modification of the oligonucleotides of the invention involves chemically linking to the oligonucleotide one or more moieties or conjugates which enhance the activity, cellular distribution or cellular uptake of the oligonucleotide. Such moieties include lipid moieties such as a cholesterol moiety, cholic acid, a thioether, a thiocholesterol, an aliphatic chain, e.g., dodecandiol or undecyl residues, a phospholipid, a polyamine or a polyethylene glycol chain, or adamantane acetic acid, a palmityl moiety, or an octadecylamine or hexylamino-carbonyl-oxycholesterol moiety.

The present invention also includes antisense compounds which are chimeric compounds. "Chimeric" antisense compounds or "chimeras," in the context of this invention, are antisense compounds, particularly oligonucleotides, which contain two or more chemically distinct regions, each made up of at least one monomer unit, i.e., a nucleotide in the case of an oligonucleotide compound. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of oligonucleotide inhibition of gene expression. Consequently, comparable results can often be obtained with shorter oligonucleotides when chimeric oligonucleotides are used, compared to phosphorothioate deoxyoligonucleotides hybridizing to the same target region. Chimeric antisense compounds of the invention may be formed as composite structures of two or more oligonucleotides, modified oligonucleotides, oligonucleosides and/or oligonucleotide mimetics as described above. Such compounds have also been referred to in the art as hybrids or gapmers.

In a further preferred embodiment of the present invention, the compounds for use in a method of treatment are compounds reducing or inhibiting the biological activity of TXNIP.

Examples of compounds capable of reducing or inhibiting the biological activity of TXNIP are (neutralizing) antibodies directed against TXNIP or fragments thereof having substantially the same binding specificity. The term "antibody", preferably, relates to antibodies which consist essentially of pooled monoclonal antibodies with different epitopic specificities, as well as distinct monoclonal antibody preparations. Monoclonal antibodies are made from an antigen containing, e.g., a fragment of TXNIP by methods well known to those skilled in the art (see e.g. (31). As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody (32). Thus, these fragments are preferred, as well as the products of a FAB or other immunoglobulin expression library. Moreover, antibodies useful for the purposes of the present invention include chimerical, single chain, and humanized antibodies.

Alternatively, preferred compounds for the purpose of the invention are inactive versions of TXNIP or nucleic acid sequences encoding inactive versions of TXNIP that can be introduced according to the approaches/vectors described above. Such inactive versions can be generated according to well known methods of mutagenesis. Such compounds can have a therapeutic effect in the human body by displacing their functionally active counterpart, in particular when applied in excess. Analyses of potentially inactive versions of TXNIP can be carried out by assaying a biological activity of TXNIP.

The present invention also relates to a method for identifying a compound reducing or inhibiting the biological activity of TXNIP and/or its expression for improving female fertility, comprising the steps of:
(a) incubating a candidate compound with a test system comprising TXNIP or its gene; and
(b) assaying a biological activity of TXNIP;
wherein an inhibition or loss of a biological activity of TXNIP, preferably compared to a test system in the absence of said test compound, is indicative of the presence of a candidate compound having the desired property.

Examples of such candidate molecules include antibodies, oligonucleotides, proteins, or small molecules. Such molecules can be rationally designed using known techniques.

Preferably, said test system used for screening comprises substances of similar chemical and/or physical properties, most preferably said substances are almost identical. The compounds which can be tested and identified according to a method of the present invention may be expression libraries, e.g., cDNA expression libraries, peptides, proteins, nucleic acids, antibodies, small organic compounds, ligands, hormones, peptidomimetics, PNAs or the like.

WO 98/25146 describes further methods for screening libraries of complexes for compounds having a desired property, especially, the capacity to agonize, bind to, or antagonize a polypeptide or its cellular receptor. The complexes in such libraries comprise a compound under test, a tag recording at least one step in synthesis of the compound, and a tether susceptible to modification by a reporter molecule. Modification of the tether is used to signify that a complex contains a compound having a desired property. The tag can be decoded to reveal at least one step in the synthesis of such a compound. Other methods for identifying compounds which interact with TXNIP or nucleic acid molecules encoding such molecules are, for example, the in vitro screening with the phage display system as well as filter binding assays or "real time" measuring of interaction.

It is also well known to the person skilled in the art, that it is possible to design, synthesize and evaluate mimetics of small organic compounds that, for example, can act as a substrate or ligand to TXNIP.

All these methods can be used in accordance with the present invention to identify a compound reducing or inhibiting the biological activity of TXNIP.

The gene encoding TXNIP can also serve as a target for the screening of inhibitors. Inhibitors may comprise, for example, proteins that bind to the mRNA of the genes encoding TXNIP, thereby destabilizing the native conformation of the mRNA and hampering transcription and/or translation. Furthermore, methods are described in the literature for identifying nucleic acid molecules such as a RNA fragment that mimics the structure of a defined or undefined target RNA molecule to which a compound binds inside of a cell resulting in the retardation of the cell growth or cell death; see, e.g., WO 98/18947 and references cited therein. These nucleic acid molecules can be used for identifying unknown compounds of pharmaceutical interest, and for identifying unknown RNA targets for use in treating a condition. These methods and compositions can be used for identifying compounds useful to reduce expression levels of TXNIP.

Furthermore, genes encoding a putative regulator of TXNIP and/or which exert their effects up- or downstream of TXNIP may be identified using, for example, insertion mutagenesis using, for example, gene targeting vectors known in the art. Said compounds can also be functional derivatives or analogues of known inhibitors. Such useful compounds can be for example transacting factors which bind to TXNIP or regulatory sequences of the gene encoding it. Identification of transacting factors can be carried out using standard methods in the art. To determine whether a protein binds to the protein itself or regulatory sequences, standard native gel-shift analyses can be carried out. In order to identify a transacting factor which binds to the protein or regulatory sequence, the protein or regulatory sequence can be used as an affinity reagent in standard protein purification methods, or as a probe for screening an expression library. The identification of nucleic acid molecules which encode polypeptides which interact with TXNIP can also be achieved, for example, as described in (33) by use of the so-called yeast "two-hybrid system". In this system TXNIP is linked to the DNA-binding domain of the GAL4 transcription factor. A yeast strain expressing this fusion polypeptide and comprising a lacZ reporter gene driven by an appropriate promoter, which is recognized by the GAL4 transcription factor, is transformed with a library of cDNAs which will express plant proteins or peptides thereof fused to an activation domain. Thus, if a peptide encoded by one of the cDNAs is able to interact with the fusion peptide comprising a peptide of TXNIP, the complex is able to direct expression of the reporter gene. In this way, TXNIP and the gene encoding TXNIP can be used to identify peptides and proteins interacting with TXNIP. It is apparent to the person skilled in the art that this and similar systems may then further be exploited for the identification of inhibitors.

The below examples explain the invention in more detail.

### Example 1

### Materials and Methods

### (A) Statistical analysis

Results are expressed as the mean ± SD if not otherwise stated. Statistical comparisons were performed using two-tailed unpaired Student's t-tests. Two-sample Kolmogorov-Smirnov tests were used to determine significance for survival curves. A statistically significant difference was defined as **P*<0.05, ***P*<0.01, ****P*<0.001.

### (B) Chemicals

Dichlorodihydrofluorescein diacetate (H₂DCF-DA) was obtained from Invitrogen (Carlsbad, CA, USA). Ionomycin was purchased from Merck (Darmstadt, Germany). All other chemicals were supplied by Sigma-Aldrich (Munich, Germany). Fluorochrome-conjugated antibodies were purchased from BD Biosciences (Heidelberg, Germany). The monoclonal antibody (OKT3) against human CD3 was prepared as described (13).

### (C) qPCR

Total RNA was isolated from primary T cells, Jurkat T cells or whole-fly lysates with the RNeasy Mini Kit (Qiagen Hilden, Germany) or TRIzol reagent (Invitrogen) according to the manufacturer's instructions. RNA was reverse transcribed and qPCR performed as described previously (15). The housekeeping gene GAPDH was used as control gene for normalization. The primer sequences were as follows: *GAPDH,* 5'-GCA AAT TCC ATG GCA CCG T-3' and 5'-TCG CCC CAC TTG ATT TTG G-3'; *TXNIP,* 5'-TCA TGG TGA TGT TCA AGA AGA TC-3' and 5'-ACT TCA CAC CTC CAC TAT C-3'; *TXN,* 5'-GAC GCT GCA GGT GAT AAA C-3' and 5'-CTG ACA GTC ATC CAC ATC TAC-3'; *ARRB1,* 5'-AGT GGC CGT GGA ACT GCC CTT CA-3' and 5'-GGA ACT TCC CGA TGC GGG GGT TC-3'; *ARRB2,* 5'-GGG CAA GCG GGA CTT CGT AGA-3' and 5'-TGC GGT CCT TCA GGT AGT CAG GG-3'.

### (D) Cell culture conditions and treatments

Jurkat J16-145 cells were derived from the human lymphoblastoid cell line Jurkat J16 (13). Jurkat cells were cultured in IMDM containing 10% FCS. Freshly isolated peripheral human T cells were cultured at a concentration of 2 x 10⁶ cells/ml in RPMI 1640 (+ l-glutamine) containing 10% FCS. Cultures were kept in a humidified 5% CO₂ incubator at 37°C. Assays were performed on primary cells that had been rested overnight.

### (E) Blood donors

T cells were isolated from the blood of healthy human donors at the age of 20 - 25 years (11 young donors) and above 55 years old (16 aged donors). Informed consent was obtained from all subjects before inclusion in the study. The study was conducted according to the ethical guidelines of the German Cancer Research Center and the Helsinki Declaration, and it was approved by the ethics committee II of the Ruprecht-Karls-University of Heidelberg, Germany.

### (F) Isolation of human peripheral T cells

Primary human T cells were purified as described (13). Purity of the prepared T cells was verified by staining with PE-conjugated anti-CD3 antibodies followed by FACS analysis.

### (G) Assessment of cell death

Cell death was assessed as the decrease in the forward-to-side scatter profile compared to living cells and recalculated to "specific cell death," as described previously (13).

### (H) Determination of ROS generation

Cells were stained with H₂DCF-DA (5 µM) for 10 min. Next, cells were divided and either stimulated with plate-bound anti-CD3 antibodies (30 µg/ml) for 1 h or left untreated. Cells were washed twice with ice-cold PBS and ROS generation was determined by FACS analysis. ROS generation was quantified as the increase in mean fluorescence intensity (MFI), calculated as reported previously (14).

### (I) Western Blot

For western blot analysis, whole cell extracts were prepared from cells or flies by lysing in ice-cold RIPA lysis buffer (50 mM Tris-HCl, pH 8.0, 120 mM NaCl, 1% NP-40, 0.5% Na-Desoxycholat, 0.1% SDS, 2 mM EDTA, 25 mM NaF, 0.2 mM NaVO4, 1 mM DTT, and complete protease inhibitor cocktail from Roche) for 30 min. Proteins were separated by SDS-PAGE and proteins were blotted onto a nitrocellulose membrane (Invitrogen Carlsbad, CA, USA) followed by blocking in 5% milk. The following antibodies were used: anti-TXNIP (1:10000, provided by T. Dick), anti-actin (1:8000, Acris Herford, Germany), anti-catalase (1:10000, Sigma-Aldrich, Munich, Germany) and anti-TXN (1:10000) (kind gift from Dr. T. Dick German Cancer Reseach Center, Heidelberg, Germany).

### (J) Generation of doxycycline (Dox)-inducible TXNIP overexpressing Jurkat T cells

Human TXNIP cDNA was amplified from the TXNIP expression vector IOH42128-pEFDEST51 (Open Biosystems Heidelberg, Germany) with the primer pair 5'-CCGGAATTCATGGTGATGTTCAAGAAGATCAAG-3' and 5'-CGGGGTACCTCACTGCACATTGTTGTTGAGG-3', and cloned into pRev-TRE-Tight (Clonetech, USA). Retroviruses were generated by transfection of Phoenix cells with pRev-TRE-TXNIP. Jurkat M2 cells, harbouring the Dox-dependent transactivator were infected and cultured in medium supplemented with 100 µg/ml hygromycin for 7 days. The resulting cells were sub-cloned twice and screened for Dox-inducible TXNIP expression by western blotting.

### (K) Lentiviral stable TXNIP knockdown

For production of lentiviral particles, HEK293T cells, pretreated with 25 µM chloroquin for 1 hour, were transfected with vectors containing the shRNA against TXNIP (Open Biosystems, Heidelberg) and a plasmid mixture for *gag, pol, env* and VSV-G for pseudotyping. 8 hours post transfection medium was replaced from packaging cells. After two days, the supernatant was passed through a 0.45 µM filter, supplemented with Polybrene (8 µg/ml). 1x10⁵ target cells were infected by spin occulation with 1 ml of viral supernatant. Stably transduced Jurkat cells were selected by puromycin resistance (1 µg/ml puromycin) and Dox-dependent shRNA expression checked by western blotting.

### (L) TXN activity assay

Analysis of TXN activity was assessed in protein lysates of primary T cells and whole flies.

### (M) Drosophila Stocks and Maintenance

For RNAi-mediated TXNIP knockdown in flies, w¹¹¹⁸; P(GD4976)v15203 female flies were obtained from the Vienna Drosophila RNAi Center (VDRC, Vienna, Austria) and crossed to *tubGal4* males provided by B. A. Edgar. Flies were reared at 25°C in standard medium.

### (N) Fertility analysis in Drosophila

For the analysis of egg laying behavior 10 newly eclosed females (on day 3 after eclosion) and five males (on day 3 after eclosion) were put together in vials and placed on agar plates containing grape juice for 24 hours. The number of eggs laid on the agar plates by each female was counted.

### (O) Lifespan studies in Drosophila

Newly eclosed flies were maintained at 25°C in vials (10 flies per vial; males and females were separated) containing standard food. Every 2-3 days flies were transferred to fresh vials and dead flies were recorded.

### Example 2

### Enhanced ROS generation in T cells of aged individuals

Previously it was shown that oxidative signals play an important role in the regulation of a T cell-mediated immune response (13-15). To analyze age-related changes of ROS generation, primary T cells from aged (> 55 years) and young (20 - 25 years old) individuals were isolated and activation-induced ROS generation was compared between these two groups. Remarkably, increased ROS production in T cells of aged donors upon T cell receptor (TCR) triggering was observed (Fig. 1a). In search for molecules regulating ROS in aged individuals, significantly enhanced expression of the negative redox regulator TXNIP in T cells of elderly individuals on mRNA as well as on the protein level was identified (Fig 1b, c). Complete activation-induced downregulation that was observed upon TCR triggering in T cells of young donors was not as efficient in T cells of the elderly. Consequently, remaining TXNIP expression could impede proper cell activation since downregulation was shown to be necessary for glucose uptake and associated cell growth (16). Consecutive investigations showed increased expression in several primary cell types, i. e. monocytes, hepatocytes and also in mesenchymal or hematopoietic stem cells of aged individuals (Figure 5a, b, c). This is consistent with a previous report showing enhanced TXNIP expression in cortices of aged individuals (17). Moreover, increased TXNIP levels were also found in CD4⁺ T cells of rheumatoid arthritis patients (Figure 5d) where the immune system is associated with an accelerated aging phenotype (18). Interestingly, analysis of the major TXNIP interaction partner, thioredoxin (TXN), revealed downregulation of TXN expression in T cells of aged individuals (Fig. 1c, d). Accordingly, a significant reduction in TXN activity in T cells isolated from aged compared to young donors was observed (Fig. 1e). These data show that high TXNIP expression is associated with a decreased anti-oxidative capacity of TXN in T cells of aged individuals.

### Example 3

### TXNIP upregulation enhances ROS generation and susceptibility to cell death

To examine the role of TXNIP in T cells use of an inducible TXNIP overexpression system in the human model T cell line Jurkat was made. TXNIP expression was induced by application of 1 µg/ml doxycycline (Dox) for 24 hours by about two-fold (Fig. 2a, b). In line with results obtained in T cells of aged individuals, induced TXNIP overexpression led to a significant increase in activation-induced ROS release upon CD3 triggering for 1 hour (Fig. 2c). For the maintenance of normal T cell homeostasis, the elimination of antigen-specific T cells after an immune response is crucial. This is achieved by triggering activation-induced cell death (AICD) *via* apoptotic pathways upon re-stimulation of the TCR (19, 20). Here, TCR-triggered oxidative signals play an important role in controlling the induction of AICD (13). Therefore, the effect of enhanced TXNIP expression on AICD was investigated. TXNIP-induced Jurkat cells were stimulated with plate-bound α-CD3 antibodies for 24 hours to induce AICD. As shown in Fig. 2d, TXNIP overexpression led to a significant increase in AICD. Together, these results show that enhanced TXNIP expression influences activation-induced ROS release and cell death in Jurkat T cells.

### Example 4

### TXNIP downregulation reduces ROS generation and enhances stress resistance

To investigate the effect of TXNIP downregulation, an inducible shRNA system was used. Incubation with Dox for 24 hours induced the expression of the TXNIP shRNA and, consequently, led to the downregulation of TXNIP mRNA and protein levels (Fig. 3a, b). It was found that loss of TXNIP expression in Jurkat T cells decreased the rate of activation-induced ROS generation upon triggering with agonistic antibodies to CD3 compared to control cells or non-induced cells (Fig. 3c). These results correspond to the data observed for primary T cells since lower TXNIP levels in young donors correlated with lower activation-induced ROS generation. Next, the effect of TXNIP downregulation on AICD induction was investigated. After induction of the specific shRNA against TXNIP by Dox treatment, Jurkat cells were stimulated with plate-bound α-CD3 antibodies for 24 hours and cell death was analyzed. In comparison to control transduced or non-induced cells, specific TXNIP downregulation led to a significant reduction in AICD (Fig. 3d) further showing that TXNIP expression is involved in cell death regulation in immune cells. To further underline these findings, cell death induction by various stimuli after specific TXNIP downregulation was analyzed using flow cytometry. In line with the results obtained with primary T cells of young *vs.* aged donors (Fig. If), specific downregulation of TXNIP led to an improved survival upon oxidative stress induction by H₂O₂ treatment (Fig. 3e). However, cell death induction by other cellular stressors like staurosporine or CD95 triggering by agonistic antibodies did not show altered levels of cell death induction whether TXNIP was downregulated or not (data not shown) indicating that proximal ROS-dependent signaling pathways upstream of CD95L transcription are affected by TXNIP. Together, these data suggest that TXNIP knockdown in Jurkat T cells did not only affect the redox equilibrium but also increased the resistance to cell death induction upon oxidative stress.

### Example 5

### TXNIP deficient flies show lifespan extension, enhanced stress resistance and increased female fertility

To analyze whether TXNIP is a marker whose expression correlates with aging or whether TXNIP is a crucial regulator of lifespan the role of TXNIP was investigated *in vivo.* The *drosophila* genome contains a homolog of human TXNIP with an overall similarity of about 47%. In *drosophila,* it was reported to play a role in nervous system development (21,22). TXNIP deficient flies were created by crossing *UAS-TXNIP* RNAi flies with *tubulin-GAL4* drivers leading to the ubiquitous downregulation of TXNIP. RNAi against TXNIP resulted in a marked reduction in TXNIP expression as determined by qPCR and Western blotting (Fig. 6a, b). Macroscopic examination of the flies showed that TXNIP deficient flies are larger (Fig. 6c). This was also reflected by higher body weight of TXNIP deficient compared to control flies (Fig. 6a). Since we observed upregulation of TXNIP expression in various human cell types it was asked whether aged control flies also show higher TXNIP levels compared to young flies. Indeed, immunoblotting of whole-fly protein extracts from control flies revealed a significant increase in TXNIP protein expression in flies at the age of 47 days compared to young flies at day 1 after birth. TXNIP deficient flies lack protein expression for lifetime confirming a highly efficient TXNIP knockdown (Fig. 6d). Next, it was investigated whether TXNIP also plays an essential role in resistance to various stress inducers, analogous to what was observed in TXNIP deficient Jurkat T cells. Therefore, TXNIP deficient flies were tested for survival under starvation and treatment with paraquat, an inducer of oxidative stress. Flies were transferred to either agar-only vials for food deprivation assays or to agar supplemented with 1 mM paraquat to test for oxidative stress resistance. TXNIP deficiency markedly increased the survival of flies under both starvation and paraquat exposure (Fig. 4b,c). Furthermore, corresponding to *in vitro* data, TXNIP deficiency caused increased TXN activity in whole-fly protein lysates (Fig. 4d), thus, affecting also the anti-oxidative capacity of *drosophila.* In order to investigate whether TXNIP deficiency is directly involved in regulation of aging lifespan in TXNIP deficient and control flies was determined. Remarkably, it was observed that genetic disruption of TXNIP significantly extended the healthy lifespan of male and female *drosophila* indicating a role for TXNIP as crucial regulator of lifespan. The median lifespan increased by 18% in female TXNIP RNAi flies, while the maximal lifespan increased by 5% (p < 0.01) in male and 13% (p < 0.001) in female RNAi flies compared to control flies, respectively (Fig.4e). In concordance with these data, previous studies also reported differences in lifespan extension between female and male organisms (23, 24). Thus, this identifies TXNIP as a direct regulator of lifespan *in vivo.* In addition, increased female fertility as determined by egg laying frequency upon deletion of TXNIP expression was observed. TXNIP deficient flies showed up to 30% increase in egg laying within the observed time period of 24 hours (Fig. 4f). These data indicate that TXNIP deficiency leads to uncoupling of lifespan and fertility as also seen upon rapamycin treatment of *drosophila* in a previous report (23). Together, the phenotypes described above demonstrate a role of TXNIP not only in the regulation of fly aging and longevity but also in the control of stress resistance and fertility.

### List of references

1. Balaban, R. S., Nemoto, S. & Finkel, T. Mitochondria, oxidants, and aging. Cell 120, 483-495, (2005).
2. Grandison, R. C., Piper, M. D. & Partridge, L. Amino-acid imbalance explains extension of lifespan by dietary restriction in Drosophila. Nature 462, 1061-1064, (2009).
3. Chen, K. S. & DeLuca, H. F. Isolation and characterization of a novel cDNA from HL-60 cells treated with 1,25-dihydroxyvitamin D-3. Biochim Biophys Acta 1219, 26-32, (1994).
4. Han, S. H. et al. VDUP1 upregulated by TGF-beta1 and 1,25-dihydorxyvitamin D3 inhibits tumor cell growth by blocking cell-cycle progression. Oncogene 22, 4035-4046, (2003).
5. Nishiyama, A. et al. Identification of thioredoxin-binding protein-2/vitamin D(3) up-regulated protein 1 as a negative regulator of thioredoxin function and expression. J Biol Chem 274, 21645-21650, (1999).
6. Junn, E. et al. Vitamin D3 up-regulated protein 1 mediates oxidative stress via suppressing the thioredoxin function. J Immunol 164, 6287-6295, (2000).
7. Chen, C. L. et al. Ceramide induces p38 MAPK and JNK activation through a mechanism involving a thioredoxin-interacting protein-mediated pathway. Blood 111, 4365-4374, (2008).
8. Chen, J., Saxena, G., Mungrue, I. N., Lusis, A. J. & Shalev, A. Thioredoxin-interacting protein: a critical link between glucose toxicity and beta-cell apoptosis. Diabetes 57, 938-944, (2008).
9. Zhou, J., Yu, Q. & Chng, W. J. TXNIP (VDUP-1, TBP-2): A major redox regulator commonly suppressed in cancer by epigenetic mechanisms. Int J Biochem Cell Biol, (2011).
10. Gokulakrishnan, K., Mohanavalli, K. T., Monickaraj, F., Mohan, V. & Balasubramanyam, M. Subclinical inflammation/oxidation as revealed by altered gene expression profiles in subjects with impaired glucose tolerance and Type 2 diabetes patients. Mol Cell Biochem 324, 173-181, (2009).
11. Zhou, R., Tardivel, A., Thorens, B., Choi, I. & Tschopp, J. Thioredoxin-interacting protein links oxidative stress to inflammasome activation. Nat Immunol 11, 136-140, (2010).
12. Fingl et al., The Pharmocological Basis of Therapeutics, Goodman and Gilman, eds. Macmillan Publishing Co., New York, pp. 1-46 ((1975).
13. Gulow, K. et al. HIV-1 trans-activator of transcription substitutes for oxidative signaling in activation-induced T cell death. J Immunol 174, 5249-5260, (2005).
14. Kaminski, M., Kiessling, M., Suss, D., Krammer, P. H. & Gulow, K. Novel role for mitochondria: protein kinase Ctheta-dependent oxidative signaling organelles in activation-induced T-cell death. Mol Cell Biol 27, 3625-3639, (2007).
15. Kaminski, M. M. et al. Mitochondrial reactive oxygen species control T cell activation by regulating IL-2 and IL-4 expression: mechanism of ciprofloxacin-mediated immunosuppression. J Immunol 184, 4827-4841, (2010).
16. Elgort, M. G., O'Shea, J. M., Jiang, Y. & Ayer, D. E. Transcriptional and Translational Downregulation of Thioredoxin Interacting Protein Is Required for Metabolic Reprogramming during G(1). Genes Cancer 1, 893-907, (2010).
17. Papadia, S. et al. Synaptic NMDA receptor activity boosts intrinsic antioxidant defenses. Nat Neurosci 11, 476-487, (2008).
18. Weyand, C. M., Fujii, H., Shao, L. & Goronzy, J. J. Rejuvenating the immune system in rheumatoid arthritis. Nat Rev Rheumatol 5, 583-588, (2009).
19. Dhein, J., Walczak, H., Baumler, C., Debatin, K. M. & Krammer, P. H. Autocrine T-cell suicide mediated by APO-1/(Fas/CD95). Nature 373, 438-441, (1995).
20. Bouillet, P. & O'Reilly, L. A. CD95, BIM and T cell homeostasis. Nat Rev Immunol 9, 514-519, (2009).
21. Mandalaywala, N. V. et al. The tumor suppressor, vitamin D3 up-regulated protein 1 (VDUP1), functions downstream of REPO during Drosophila gliogenesis. Dev Biol 315, 489-504, (2008).
22. Chang, S., Mandalaywala, N. V., Snyder, R. G., Levendusky, M. C. & Dearborn, R. E., Jr. Hedgehog-dependent downregulation of the tumor suppressor, vitamin D3 up-regulated protein 1 (VDUP1), precedes lamina development in Drosophila. Brain Res 1324, 1-13, (2010).
23. Bjedov, I. et al. Mechanisms of life span extension by rapamycin in the fruit fly Drosophila melanogaster. Cell Metab 11, 35-46, (2010).
24. Fontana, L., Partridge, L. & Longo, V. D. Extending healthy life span--from yeast to humans. Science 328, 321-326, (2010).
25. Watanabe, R., Nakamura, H., Masutani, H. & Yodoi, J. Anti-oxidative, anti-cancer and anti-inflammatory actions by thioredoxin 1 and thioredoxin-binding protein-2. Pharmacol Ther 127, 261-270, (2010).
26. Zimmermann et al., (1994) Neuron 12, 11-24.
27. Vidal et al., (1990) EMBO J. 9, 833-840.
28. Mayford et al., (1995), Cell 81, 891-904.
29. Pinkert et al., (1987) Genes & Dev. 1, 268-76.
30. Nielsen et al., Science 254 (1991), 1497-1500.
31. Köhler et al., Nature 256 (1975), 495.
32. Wahl et al., J. Nucl. Med. 24: 316-325 (1983).
33. Scofield, Science 274 (1996), 2063-2065.

## Claims

1. A compound capable of reducing or inhibiting (a) the biological activity of thioredoxin-interacting protein (TXNIP) or (b) the expression of the gene encoding TXNIP for use in a method of improving female fertility,
**characterized in that** said compound is
(i) an antisense oligonucleotide or shRNA reducing or inhibiting the expression of the gene encoding TXNIP, or
(ii) an antibody directed against TXNIP or a fragment thereof,
or
(iii) an inactive version of TXNIP or nucleic acid sequences encoding an inactive version of TXNIP.

2. A method for identifying a compound that improves female fertility by reducing or inhibiting the biological activity of TXNIP or the expression of the gene encoding TXNIP, comprising the steps of:
(a) incubating a candidate compound with a test system comprising TXNIP or the gene encoding TXNIP; and
(b) assaying a biological activity of TXNIP;
wherein an inhibition or loss of the biological activity of TXNIP is indicative of the presence of a candidate compound having the desired property.

3. The method of claim 2, wherein the inhibition or loss of the biological activity of TXNIP is determined by comparison to a test system **characterized by** the absence of said test compound.

## Patentansprüche

1. Verbindung, mit der (a) die biologische Aktivität des mit Thioredoxin interagierenden Proteins (TXNIP) oder (b) die Expression des Gens, das TXNIP kodiert, reduziert oder gehemmt werden kann, zur Verwendung in einem Verfahren zum Verbessern der weiblichen Fruchtbarkeit,
**dadurch gekennzeichnet, dass** die Verbindung
(i) ein Antisense-Oligonukleotid oder eine shRNA ist, das bzw. die die Expression des Gens, das TXNIP kodiert, reduziert oder hemmt, oder
(ii) ein Antikörper ist, der gegen TXNIP oder ein Fragment davon gerichtet ist, oder
(iii) eine inaktive Version von TXNIP oder Nukleinsäuresequenzen ist, die für eine inaktive Version von TXNIP kodieren.

2. Verfahren zum Identifizieren einer Verbindung, die die weibliche Fruchtbarkeit dadurch verbessert, dass sie die biologische Aktivität von TXNIP oder die Expression des Gens, das TXNIP kodiert, reduziert oder hemmt, umfassend die folgenden Schritte:
(a) Inkubieren einer Kandidatenverbindung mit einem Testsystem, umfassend TXNIP oder das Gen, das TXNIP kodiert; und
(b) Untersuchen einer biologischen Aktivität von TXNIP;
wobei eine Hemmung oder ein Verlust der biologischen Aktivität von TXNIP das Vorhandensein einer die gewünschte Eigenschaft aufweisenden Kandidatenverbindung anzeigt.

3. Verfahren nach Anspruch 2, wobei die Hemmung oder der Verlust der biologischen Aktivität von TXNIP durch Vergleich mit einem Testsystem bestimmt wird, **gekennzeichnet durch** das Fehlen der Testverbindung.

## Revendications

1. Composé à même de réduire ou d'inhiber (a) l'activité biologique de la protéine interagissant avec la thiorédoxine (TXNIP) ou (b) l'expression du gène codant pour TXNIP destiné à être utilisé dans un procédé pour améliorer la fertilité féminine, **caractérisé en ce que** ledit composé est
(i) un oligonucléotide anti-sens ou ARNsh réduisant ou inhibant l'expression du gène codant pour TXNIP ou
(ii) un anticorps dirigé contre TXNIP ou un fragment de ce dernier, ou
(iii) une version inactive de TXNTP ou des séquences d'acide nucléique codant pour une version inactive de TXNIP.

2. Procédé pour identifier un composé qui améliore la fertilité féminine en réduisant ou inhibant l'activité biologique de TXNIP ou l'expression du gène codant pour TXNIP, comprenant les étapes consistant à:
(a) incuber un composé candidat par un système de test comprenant TXNIP ou le gène codant pour TXNIP; et
(b) tester une activité biologique de TXNIP;
dans lequel une inhibition ou une perte de l'activité biologique de TXNIP est une indication de la présence d'un composé candidat présentant la propriété souhaitée.

3. Procédé selon la revendication 2, dans lequel l'inhibition ou la perte de l'activité biologique de TXNIP est déterminée par comparaison avec un système de test **caractérisé par** l'absence dudit composé de test.
